(19) Europäisches Patentamt / European Patent Office / Office européen des brevets

(11) **EP 4 434 443 A1**

(12) **EUROPEAN PATENT APPLICATION**

(43) Date of publication:
**25.09.2024 Bulletin 2024/39**

(21) Application number: **23163671.3**

(22) Date of filing: **23.03.2023**

(51) International Patent Classification (IPC):
**A61B 5/00** (2006.01)    **G01F 1/663** (2022.01)
**A61B 8/08** (2006.01)

(52) Cooperative Patent Classification (CPC):
**A61B 5/4288; A61B 5/4312; A61M 1/062;**
**G01F 1/661; G01F 1/663; G01F 25/10;**
A61M 2205/3306; A61M 2205/70

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **SEGAAR, Lucja Elzbieta**
**Eindhoven (NL)**
• **GERHARDT, Lutz Christian**
**Eindhoven (NL)**
• **MENA BENITO, Maria Estrella**
**Eindhoven (NL)**
• **JOHNSON, Mark Thomas**
**Eindhoven (NL)**
• **KOOIJMAN, Gerben**
**Eindhoven (NL)**

(74) Representative: **Philips Intellectual Property &**
**Standards**
**High Tech Campus 52**
**5656 AG Eindhoven (NL)**

(54) **CALIBRATING DUCT PARAMETER MEASUREMENTS**

(57) Proposed concepts thus aim to provide schemes, solutions, concept, designs, methods and systems pertaining to calibrating a duct parameter measurement of a milk duct of a breast of a subject. In particular, embodiments aim to provide a method for calibrating a duct parameter measurement of a milk duct of a subject by using a detected MER parameter value, such as the peak of the MER cycle, to calibrate the duct parameter value of the milk duct of the subject. In this way, duct parameter values may be accurately compared over time, or between breasts, to monitor duct health and to assess the risk of the milk duct clogging.

FIG. 1

EP 4 434 443 A1

**Description**

FIELD OF THE INVENTION

This invention relates to the field of calibrating duct parameter measurements, and in particular to the field of calibrating a duct parameter measurement of a milk duct of a subject.

BACKGROUND OF THE INVENTION

Breastfeeding is crucial for healthy development of a newborn baby. Indeed, the best source of nutrition for a baby is human milk given by a breastfeeding mother. The milk contains antibodies which help protect against many common childhood and future illnesses. The World Health Organization recommends to exclusively breastfeed for at least six months, meaning no other foods or liquids are provided.

During breastfeeding, the infant interacts with the nipple applying both vacuum cycles and tongue pressures to extract milk. Moreover, the infant stimulates the nipple and areola complex that leads to release of oxytocin and ejection of milk. The combination of the applied vacuum, the contracting response of the cells around the ducts (myoepithelial cells) to oxytocin release and the pressure of milk flowing through the ducts leads to opening of the milk ducts and release of milk. In this process, the milk ducts show highly dynamic behavior, going from a collapsed to a swollen milk-filled state.

However, breastfeeding is often halted due to breastfeeding related breast pain and breast traumas caused by the act of breastfeeding and/or milk expression itself. Pain and trauma are among the main causes of weaning from breastfeeding.

For example, mastitis is a common complication occurring during breastfeeding, being a painful inflammation of the breast tissue. It is most often caused by clogged milk ducts that lead to milk stasis (remaining milk in the breast after a feed giving a restricted milk flow), sometimes in combination with infectious bacteria. Clogged milk ducts refer to milk ducts with some obstruction, e.g. caused by milk clutters. This can lead to nipples that are painful, feel warm, cause discomfort and, as stated above, lead to the development of mastitis.

Some signs can be predictive of clogged ducts, including localized redness of the skin, a hard or semi-hard lump on the breast, and a feeling of pain during milk ejection reflex.

However, due to the many various symptoms which can result from breastfeeding (like a damaged nipple, blebs, abscesses and more) mothers often find it difficult to recognize and identify the right condition and, as consequence, do not timely apply a suitable treatment for preventing the development of more serious conditions, such as mastitis due to clogged ducts.

In clinical breastfeeding research, ultrasound-based imaging technologies are used to image milk ducts. This imaging method is difficult to translate to a home solution. Also, ultrasound imaging requires a coupling between the ultrasound transducer and the tissue, e.g., using an ultrasound gel, which is inconvenient and also not desirable as it may end up in the milk. Furthermore, ultrasound does not have a high enough resolution to see narrower sections of the ducts, duct endings being of the order of 0.07 mm in size.

Currently, there are no consumer-friendly solutions for early detection of clogged ducts. The only methods relate to visual or physical judgment of the mother herself or a trained professional. Those methods, however, come often late or are very subjective.

A technology to detect clogged milk ducts early, and which is suitable for consumer use, would be of interest. This would allow mothers to clear clogged ducts in time to prevent pain, the development of mastitis and ultimately to prevent weaning from breastfeeding.

In order to detect clogged milk ducts, however, a method is needed for calibrating milk duct parameter measurements. Due to a large variation (up to a factor of 3 to 4) in ductal size and the milk flow within a breastfeeding session or over longitudinal sessions, there is a need to calibrate duct dilation or milk flow measurements in order to ensure the accurate detection of clogging of milk ducts.

Milk ejection reflex (MER) is crucial in successful lactation. As the milk flows through the mammary gland in response to MER, the pressure within the milk ducts increases. As the milk pressure increases, there is a corresponding transient increase in duct diameters demonstrated by ultrasound monitoring of milk ducts.

Ejection of milk after MER takes place 30 seconds to 1 minute after release of oxytocin and typically lasts between 45 seconds and 4 minutes. Milk ejection reflex occurs several times during a feeding or expression session with a few minutes in between. The first MER is usually the strongest and releases significantly more milk than subsequent milk ejections, regardless of vacuum level applied. Successive ejections are less strong and rarely directly perceived.

During breast feeding or milk expression, MER occurs on average 4 to 5 times, which allows the entire mammary system to be activated more than once to efficiently remove all the milk from the alveoli and empty the breast. Moreover, MER has been shown to present the same characteristics for an individual, for instance, the number of MER cycles in an individual's breastfeeding session.

As the milk flows through the breast in response to MER, the pressure within the milk ducts increases and dilation of the duct is observed. It has been found that the diameter of the milk ducts starts to increase sometime after nipple stimulation, on average 50 seconds.

Due to the dramatic changes in milk duct geometry during an MER cycle, the difference between ductal measurements taken at random within a breastfeeding session could be very large, up to a factor of 3 to 4, and therefore this natural variation needs to be considered for a reliable assessment of duct clogging.

Furthermore, ultrasound imaging has shown independence of the peak duct dilation from the amount of milk available in the breast, which suggests that each duct has a maximum diameter. Dilation of the milk ducts and intraductal pressure peaks have also been shown to be higher for the first ejection reflex as compared to successive ejections of milk.

There is therefore a need for a method for calibrating a duct parameter measurement of a milk duct of a subject.

SUMMARY OF THE INVENTION

The invention is defined by the claims.

According to examples in accordance with an aspect of the invention, there is provided a method for calibrating a duct parameter measurement of a milk duct of a breast of a subject.

The method comprises: detecting at least one milk ejection reflex (MER) parameter value of at least one MER cycle of the subject; determining at least one duct parameter value of a milk duct of the subject; and calibrating the determined at least one duct parameter value based on the detected at least one MER parameter value to generate at least one calibrated duct parameter value.

Proposed concepts thus aim to provide schemes, solutions, concept, designs, methods and systems pertaining to calibrating a duct parameter measurement of a milk duct of a breast of a subject. In particular, embodiments aim to provide a method for calibrating a duct parameter measurement of a milk duct of a breast of a subject by using a detected MER parameter value, such as the peak of the MER cycle, to calibrate a duct parameter value of the milk duct of the subject. In this way, duct parameter values may be accurately compared longitudinally, i.e. over time, or between breasts. This may allow the accurate and reliable monitoring of duct health and the assessment of the risk of milk duct clogging.

In other words, it is proposed that by calibrating a duct parameter value using an MER parameter value, the duct parameter value can be calibrated, standardized, normalised, or otherwise modified to facilitate informative and fair comparing to other calibrated duct parameter values. As an example, a duct parameter value may be duct diameter or milk flow rate. As an example, an MER parameter value may be whether the MER cycle is the first in a breastfeeding session or it may be the identification of the peak of the MER cycle. Identification of the peak of the MER cycle may comprise determining its time of occurrence. As duct parameter values, such as duct diameter, can vary dramatically over an MER cycle, the context of the duct parameter value needs to be taken into account to ensure that when comparing to other values, the comparison is useful and informative. A comparison between calibrated values can then be used to monitor duct health and potentially to indicate a risk of the milk duct clogging or being clogged.

By detecting one or more parameters of an MER cycle, for instance the start of the MER cycle and the peak of the MER cycle, one or more duct parameter values can be calibrated. For instance, the milk flow rate both at the start of the MER cycle and at the peak of the MER cycle can be used to calculate a calibrated milk flow difference. This calibrated milk flow difference value can then be compared across different breastfeeding sessions for the same subject to monitor their ducts. If this value substantially drops, this may then indicate milk duct clogging.

In a simple example, after detecting the peak of the first MER cycle in a breastfeeding or milk expression session, the duct diameter or milk flow rate can be determined at that same exact point in each breastfeeding session and thus can be fairly compared to one another. This comparison may, for example, be for the same breast over time or between a subject's right and left breasts in the same session. If the values are different from one another by more than a threshold amount, this might then indicate a risk of milk duct clogging. Thus, a way to reliably calibrate and personalise duct parameter values is provided, and this can be put to use in the detection of milk duct clogging, or a risk of milk duct clogging.

In summary, because of the variation in milk flow caused by an MER cycle, and the associated large variation in ductal size/diameter within a breastfeeding session or different sessions over time (weeks/months of lactation), there is a need to account for these variations to enable reliable early detection of duct blockage. It is thus beneficial to standardize or calibrate the duct and/or milk flow measurements so that they may be used as metrics reliably indicative of clogging or early detection of clogging.

Therefore, within this invention, there are proposed optical and/or non-optical methods to detect milk ejection reflex (MER) parameter values and to use this information to calibrate and time-correlate milk ejection events with either milk duct geometry changes or flow changes as indicators of duct clogging

The inventors have realised that in order to correctly and reliably identify early signs of duct clogging or blockage, it is necessary to take into account the phase of the milk ejection reflex e.g., whether the measurement is taken at the peak of an MER or between MERs, and whether at the first peak or at subsequent peaks. In such a manner, it becomes possible to calibrate and/or compare longitudinal measurements with higher accuracy by considering, for example: a longitudinal comparison of measurements at the same point in the MER patterns; by choosing a particular point on the MER pattern to carry out measurements, especially choosing measurements at the peak of the MER (i.e., maximum duct diameter or milk flow), more preferably the peak of the first MER; by a calibration of single measurements considering the point of the MER pattern at which measurement is taken; or by calibration using a well-defined time interval/period of milk ejection, such as time to peak duct diameter of the first MER cycle or the interval between the start and end of an MER cycle.

This invention may be of particular use implemented into a breast pump or a nipple shield, so that the health of milk ducts of a breast of a subject may be monitored as part of a daily routine in a non-intrusive way.

Ultimately, an improved method calibrating a duct parameter measurement of a milk duct of a breast of a subject may be supported by the proposed concept(s).

In some embodiments, the at least one MER parameter value may comprise at least one of: the peak of the MER cycle; the duration of the MER cycle; whether the MER cycle is the first in a breastfeeding or milk expression session; the start of the MER cycle; the end of the MER cycle; the duration from the start to the peak of the MER cycle; the duration from the peak to the end of the MER cycle.

By taking into account the specific point or points of the milk ejection reflex cycle that the duct parameter value is related to, the duct parameter value can be calibrated. The peak of an MER cycle can be used, for instance, to calibrate a maximum duct diameter value as it is a very specific and identifiable spot within an MER cycle. It can therefore be used to ensure that comparisons between duct diameter values are reliable and that any variation is not due to fluctuations within the MER cycle itself. The duration of an MER cycle can be used to determine, for instance, the total milk volume expressed over a single MER cycle. Whether the MER cycle is the first in a breastfeeding or milk expression session can be used to help calibrate the duct parameter value as the first MER cycle is usually the strongest and releases significantly more milk than subsequent milk ejections. Therefore, comparing the peak of a first MER cycle to the peak of a second MER cycle in different breastfeeding sessions would not be a particularly informative comparison. The start of an MER cycle can be used to determine, for instance, the time to milk ejection. The end of an MER cycle can be used to determine, for instance, a duct parameter value between MER cycles in a single breastfeeding session, e.g. at the end of the first MER cycle in a session, before the second. The duration from the start to the peak of an MER cycle can be used to determine, for instance, the leading edge of the duct diameter. The duration from the peak to the end of an MER cycle can be used to determine, for instance, the falling edge of the duct diameter.

In some embodiments, detecting at least one MER parameter value of at least one MER cycle of the subject may comprise obtaining measurements of the at least one MER cycle of the subject from an optical imaging system. By obtaining measurements using an optical imaging system, MER parameters can be detected non-invasively and in a way that may seamlessly integrate into an efficient breastfeeding workflow. This is in contrast to intraductal pressure measurements or the use of electrodes or ultrasound gel. The optical imaging system may be a stand-alone unit or, for instance, integrated into a system such as a breast pump for carrying out any herein disclosed method.

In some embodiments, determining the at least one duct parameter value may be based on the obtained measurements of the at least one MER cycle of the subject. The optical imaging system may simultaneously measure milk flow and duct geometry parameter values. Alternatively, the optical imaging system may only measure one parameter at a time. Milk ducts can be identified in images obtained from the optical imaging system and their geometry can thus be analysed. For example, milk duct diameter can be measured as a simple linear dimension within a captured image. The change in geometry during a breastfeeding or milk expression session, which can be determined from the obtained images, can then be used to help detect milk duct blockages or partial blockages.

In some embodiments, the at least one duct parameter value may comprise at least one of: milk flow rate; maximum milk flow rate; milk flow volume; total milk volume; duct diameter; change of duct diameter; rate of change of duct diameter; rate of change of duct diameter from the start to the peak of the MER cycle; duct volume; change of duct volume; and rate of change of duct volume. These particular duct parameter values have been found to be particularly beneficial parameters for monitoring the health of milk ducts and for determining whether they are already clogged or there is a risk of milk duct clogging.

In some embodiments, the at least one calibrated duct parameter value may be indicative of at least a partial clogging of the milk duct of the subject. In this way, partial clogging of the milk duct of the subject may be determined from the calibrated value and the subject may be alerted that their milk duct is partially clogged and at risk of substantial or full clogging.

In some embodiments, the method may further comprise determining a risk value indicating a risk of the milk duct of the subject clogging based on the at least one calibrated duct parameter value. After calibrating the milk duct parameter value, a risk of the milk duct clogging may be determined. For instance, whether the milk duct is partially, substantially, or fully clogged may be determined. The risk value may indicate a probability or likelihood of the milk duct clogging in the near future. The risk value may indicate a probability or likelihood of the milk duct being partially or fully clogged at the time the duct parameter value was determined. The subject may then be informed of this information.

In some embodiments, determining a risk value may comprise comparing the at least one calibrated duct parameter value to a threshold value, and determining the risk value based on the comparison result. The use of a threshold may provide an efficient way to determine whether the milk duct is clogged or at risk of clogging, either partially, substantially, or fully.

In some embodiments, the threshold value may be determined based on at least one past calibrated duct parameter value of the subject's milk duct. The use of a past calibrated duct parameter value of the subject's milk duct may provide a personalized threshold that takes account of the fact that milk duct parameters, such as milk flow rate or duct diameter, can substantially vary between different subjects. In other words, the determining of a risk of milk duct clogging can be personalized to individual subjects based off their past calibrated duct parameter values. Past calibrated duct parameter values may be associated with individual milk ducts of the subject, and thus thresholds may be personalized to the exact milk duct of the subject. Alternatively, the threshold may be based on at least one past calibrated duct parameter value of any of the subject's milk ducts.

In some embodiments, the threshold value may be determined based on at least one calibrated duct parameter value of a different milk duct of the subject. The use of a calibrated duct parameter value of a different milk duct of the subject to help inform the threshold value may allow variation between ducts in a single breast or between breasts to be indicative of milk duct clogging.

In some embodiments, the milk duct of the subject may be in a different breast to the different milk duct. The use of a calibrated duct parameter value of a different milk duct of the subject to help inform the threshold value may allow variation between breasts to be indicative of milk duct clogging. For instance, calibrated duct parameter values may be determined for the left and right breasts simultaneously, and this information may be used to help determine whether one breast is more at risk of clogging than the other. Alternatively, calibrated duct parameter values may be determined for the left and right breasts at different times.

In some embodiments, the at least one duct parameter value may comprise both change of duct diameter and maximum flow rate. The use of these two parameters, and in particular the relationship between them, has been found to be particularly beneficial in assessing the risk of milk duct clogging.

In some embodiments, the method may further comprise generating a display control signal, the display control signal describing the determined risk. This may provide a way of presenting to the subject the risk of one of their milk ducts being clogged. For instance, the display control signal may comprise feedback that the milk duct most recently measured is clogged or at risk of clogging. In another example, the display control signal may comprise feedback indicating an area at which a milk-blocked duct has been identified. This may enable the subject to take corrective curative action.

According to another aspect of the invention, there is provided a computer program comprising code means for implementing the method of any herein disclosed method when said program is run on a processing system.

According to another aspect of the invention, there is provided a system for calibrating a duct parameter measurement of a milk duct of a subject. The system comprising a processor arrangement configured to: detect at least one milk ejection reflex (MER) parameter value of at least one MER cycle of the subject; determine at least one duct parameter value of a milk duct of the subject; and calibrate the determined at least one duct parameter value based on the detected at least one MER parameter value to generate at least one calibrated duct parameter value.

In some embodiments, the system may further comprise an optical imaging unit, configured to obtain measurements of the at least one MER cycle of the subject, wherein detecting the at least one MER parameter value is based on the obtained measurements. The system does not require an optical imaging unit, as the system may receive information about at least one MER cycle of the subject from a stand-alone unit, such as a stand-alone optical imaging unit or from a bottle unit that, for example, monitors the rate of filling. Integrating an optical imaging unit into the system, however, may provide a comprehensive system capable of carrying out any herein disclosed method.

According to another aspect of the invention, there is provided a breast pump comprising any herein disclosed system.

Thus, there may be proposed concepts for calibrating a duct parameter measurement of a milk duct of a subject, and this may be done based on detected MER parameter values and determined duct parameter values.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for calibrating a duct parameter measurement of a milk duct of a subject according to a proposed embodiment;

Fig. 2 is a diagram showing how the diameter of a milk duct of a subject changes over time during a breastfeeding session;

Fig. 3 is a more in-depth flow diagram of a method for calibrating a duct parameter measurement of a milk duct of a subject according to a proposed embodiment;

Fig. 4 is a diagram showing the progression of relative levels of duct clogging for two milk ducts of a subject respectively;

Fig. 5 is a diagram showing a progressive dilation of duct lumen behind a clogged duct at a fixed position;

Fig. 6 is a diagram showing dilation of duct lumen behind a clogged duct;

Fig. 7 is a simplified block diagram of a system for calibrating a duct parameter measurement of a milk duct of a subject according to a proposed embodiment;

Fig. 8 is a simplified block diagram of a breast pump comprising a system for calibrating a duct parameter measurement of a milk duct of a subject according to a proposed embodiment; and

Fig. 9 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to calibrating a duct parameter measurement of a milk duct of a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for calibrating a duct parameter measurement of a milk duct of a subject. This can be achieved by detecting an MER parameter value of an MER cycle of the subject, and then using this MER parameter value to calibrate a determined duct parameter value of the subject's milk duct.

Proposed concepts thus aim to take into account the context in which a duct parameter value of a milk duct of the subject was determined, and by doing so to calibrate it so that it may be more usefully compared to other calibrated duct parameter values to potentially indicate whether a milk duct is clogged or at risk of clogging.

In other words, in order to correctly and reliably identify early signs of duct clogging or blockage, it is necessary to take into account the phase of the milk ejection reflex e.g., whether a measurement is taken at the peak of an MER cycle or between MER cycles, and whether at the first peak or at subsequent peaks. In such a manner, it becomes possible to calibrate and/or compare longitudinal measurements with higher accuracy.

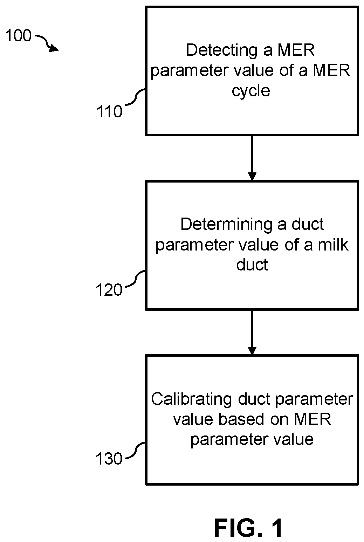

Referring now to Fig. 1, there is depicted a simplified flow diagram of a method 100 for calibrating a duct parameter measurement of a milk duct of a subject according to a proposed embodiment.

The method 100 begins with the step 110 of detecting at least one milk ejection (MER) parameter value of at least one MER cycle of the subject.

The at least one MER parameter value comprises at least one of: the peak of the MER cycle; the duration of the MER cycle; whether the MER cycle is the first in a breastfeeding or milk expression session; the start of the MER cycle; the end of the MER cycle; the duration from the start to the peak of the MER cycle; the duration from the peak to the end of the MER cycle.

By taking into account the specific point or points of the milk ejection reflex cycle that the duct parameter value is related to, the duct parameter value can be calibrated. The peak of an MER cycle can be used, for instance, to calibrate a maximum duct diameter value as it is a very specific and identifiable spot within an MER cycle, and so can be used to ensure that comparison between duct diameter values is reliable and that any variation is not due to fluctuations within the MER cycle itself. The duration of an MER cycle can be used to determine, for instance, the total milk volume expressed over a single MER cycle as well as other relevant duct parameter values such as milk flow or milk flow rate. Whether the MER cycle is the first in a breastfeeding or milk expression session can be used to help calibrate the duct parameter value as the first MER cycle is usually the strongest and releases significantly more milk than subsequent milk ejections. Therefore, comparing the peak of a first MER cycle to the peak of a second MER cycle in different breastfeeding sessions would not be a particularly informative comparison. The start of an MER cycle can be used to determine, for instance, the time to milk ejection. The end of an MER cycle can be used to determine, for instance, a duct parameter value between MER cycles in a single breastfeeding session, e.g. at the end of the first MER cycle in a session, before the second. The duration from the start to the peak of an MER cycle can be used to determine, for instance, the leading edge of the duct diameter. The duration from the peak to the end of an MER cycle can be used to determine, for instance, the falling edge of the duct diameter.

Any optical or non-image-based means may be utilised to detect an MER cycle of the subject using technologies and solutions such as, for instance, validated detection and measurement techniques. These may be, for example, maternal circulating oxytocin, intraductal pressure, ultrasound imaging, milk flow rates, bioimpedance analysis, and thermal imaging.

In step 120, at least one duct parameter of a milk duct of the subject is determined. The at least one duct parameter value comprises at least one of: milk flow rate; maximum milk flow rate; milk flow volume; total milk volume; duct diameter; change of duct diameter; rate of change of duct diameter; rate of change of duct diameter from the start to the peak of the MER cycle; duct volume; change of duct volume; and rate of change of duct volume. These particular duct parameter values have been found to be particularly beneficial parameters for monitoring the health of milk ducts and for determining whether they are clogged or at risk of clogging.

The at least one duct parameter value may comprise both change of duct diameter and maximum flow rate. The use of these two parameters, and in particular the relationship between them, has been found to be particularly beneficial in assessing the risk of a milk duct clogging.

Software and/or algorithms may be used to determine the at least one duct parameter value of a milk duct of the subject. For example, a processor may execute an algorithm to extract duct diameter, duct volume and/or milk flow rate/volume from measurements, such as images. The milk duct diameter increases as the pressure in the milk duct rises and subsequently decreases as the pressure decreases due to milk outflow during an MER cycle. Software and/or algorithms may establish a relationship between the changes in duct diameter and a maximum milk flow rate to be used as a reference calibration or standardized time point. By evaluating duct diameter and/or milk flow patterns over time during certain time intervals, a method may be provided that is able to establish and assess the existence of clogged ducts by linking and comparing a reference or threshold duct diameter, peak flow, rate of change of diameter, or rate of change of duct diameter in the leading edge.

In step 130, the determined at least one duct parameter value is calibrated based on the detected at least one MER parameter value to generate at least one calibrated duct parameter value. Calibrating may also be referred to as standardizing, normalising, or otherwise modifying. Calibrating involves considering the phase of the MER cycle at which the duct parameter was determined. For instance, a milk flow rate may be calibrated by relating it to a particular point within the MER cycle from which it was measured. The particular point may be the peak of the MER cycle. As an example, calibrating may also comprise adding up the milk flow over an entire MER cycle to generate a calibrated total milk volume expressed over the MER cycle. As another example, calibrating may also comprise subtracting the duct diameter at the start of an MER cycle from the duct diameter at the peak of the MER cycle in order to generate a calibrated change in duct diameter value.

For instance, software and/or algorithms may be used to calibrate a duct parameter value to a known point of an MER cycle.

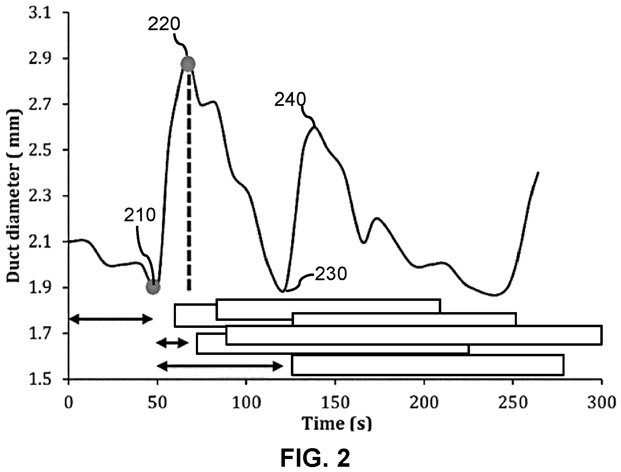

Referring now to Fig. 2, there is depicted a diagram showing how the diameter of a milk duct of a subject changes over time during an breastfeeding session. The first MER cycle in the breastfeeding session is from point 210 to point 230, with point 220 being the peak of the first MER cycle. Point 230 is also the start of the second MER cycle in the session, with point 240 being the peak of the second MER cycle.

The duration from the start of the session to point 210 is known as time to milk ejection. The duration from point 210 to point 220 is known as time to peak. The duration from point 210 to point 230 is known as the duration of a MER cycle. The duration from the peak to the end of the MER cycle is the duration from point 220 to point 230. The graph line from point 210 to point 220 as known as the leading edge. The graph line from point 220 to point 230 is known as the falling edge. The maximum duct diameter is found at the peak of the first MER cycle, i.e. point 220. The change of duct diameter may be the change between the duct diameter at point 220 and point 210 or 230, depending on which is lower.

A time integration of area under the curve between points 210 and 230 can provide a duct volume (lumen) parameter. If the y-axis were milk flow rate instead, the total milk volume expressed over the first MER cycle could be calculated by a time integration of area under the curve between points 210 and 230.

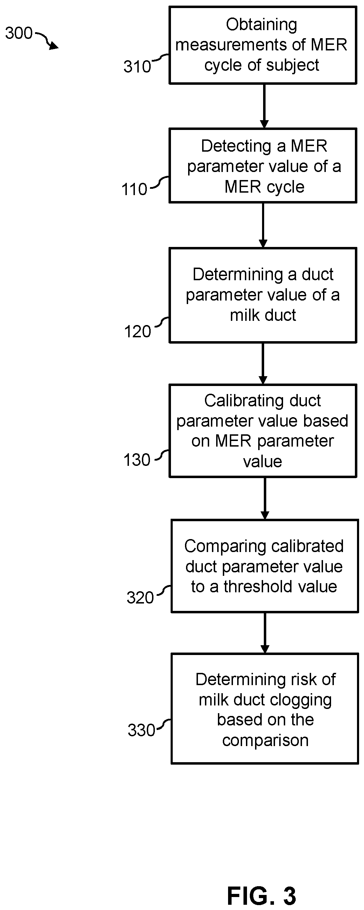

Referring now to Fig. 3, there is depicted a more in-depth flow diagram of a method 300 for calibrating a duct parameter measurement of a milk duct of a subject according to a proposed embodiment.

Steps 110, 120, and 130 are substantially the same as have been described in relation to the method 100 in Fig. 1.

In step 310, measurements of the at least one MER cycle of the subject are obtained from an optical imaging system. By obtaining measurements using an optical imaging system, MER parameters can be detected non-invasively and in a way that may seamlessly integrate into an efficient breastfeeding workflow. This is in contrast to intraductal pressure measurements or the use of electrodes or ultrasound gel. The optical imaging system may be a stand-alone unit or, for instance, integrated into a system such as a breast pump for carrying out any herein disclosed method. The optical imaging system can detect the details of an individual's MER cycles and the dynamics of milk removal. Preferably, the optical imaging system is used to monitor and measure changes in ductal diameter at the moment of milk ejection.

The optical imaging system may be an OCT doppler flow imaging unit, a transmission imaging system, or an OCT system with static optics, such that a pressure drop or increase in milk flow is measured.

In other words, in this embodiment the use of an optical (sub-surface) imaging system is proposed for image-based measurement of milk flow (rate, volume) and duct geometry (diameter, volume) to calibrate and time-correlate milk ejection events with either milk duct geometry changes or flow changes as indicators for duct clogging. The image-based measurement of milk flow and duct geometry may be simultaneous or combined, or separate.

The at least one duct parameter value is also based on the obtained measurements of the at least one MER cycle of the subject. The optical imaging system may simultaneously measure milk flow and duct geometry parameter values, or alternatively only one parameter at a time. Milk ducts can be identified in images obtained from the optical imaging system and their geometry can thus be analysed. For example, milk duct diameter can be measured as a simple linear dimension within a captured image. The change in geometry during a breastfeeding or milk expression session, which can be determined from the obtained images, can then be used to help detect milk duct blockages or partial blockages.

In this embodiment, the at least one calibrated duct parameter value is indicative of at least a partial clogging of the milk duct of the subject. In this way, partial clogging of the milk duct of the subject may be determined from the calibrated value and the subject may be alerted that their milk duct is partially clogged and at risk of substantial or full clogging.

Calibration of the duct parameter value may be done by normalizing or standardizing the respective duct parameter value or values to the first MER cycle/event of a first lactiferous duct of a first feeding or pumping session. This allows the reliable assessment of temporal changes of the overall duct lumen occurring during breast feeding or breast pumping.

In step 320, the at least one calibrated duct parameter value is compared to a threshold value. The use of a threshold provides an efficient way to determine whether the milk duct is clogged or at risk of clogging, either partially, substantially, or fully.

In step 330, a risk value is determined indicating a risk of the milk duct of the subject clogging based on the comparison, which itself is based on the at least one calibrated duct parameter value. After calibrating the milk duct parameter value, a risk of the milk duct clogging can be determined. For instance, whether the milk duct is partially, substantially, or fully clogged can be determined. The risk value may indicate a probability of the milk duct clogging in the near future. The risk value may indicate a probability of the milk duct being partially or fully clogged at the time the duct parameter value was determined. The subject may then be informed of this information.

In this embodiment, the threshold value is determined based on at least one past calibrated duct parameter value of the subject's milk duct. The use of a past calibrated duct parameter value of the subject's milk duct provides a personalized threshold that takes account of the fact that milk duct parameters, such as milk flow rate or duct diameter, can substantially vary between different subjects. In other words, the determining of a risk of milk duct clogging can be personalized to individual subjects based off their past calibrated duct parameter values. Past calibrated duct parameter values may be associated with individual milk ducts of the subject, and thus thresholds may be personalized to the exact milk duct of the subject. Alternatively, the threshold may be based on at least one past calibrated duct parameter value of any of the subject's milk ducts. As an example, the threshold may be based on at least two past duct parameter values, for instance, their average. Comparing may comprise determining if a calibrated duct parameter value exceeds the threshold value by a certain percentage, for instance, 10%, 20%, 30%, 40%, 50%, 60%, 70%, 80%, 90%, 100%.

In a simple embodiment, an absolute level or percentage of clogging of a duct may be written as a function of longitudinal first MER cycle and expressed mathematically as:

$$AC = 1 - \frac{DPV(MER_{1,t})}{DPV(MER_{1,1})} \qquad (1)$$

AC stands for absolute clogging. DPV stands for a duct parameter value. $MER_{1,1}$ is the first MER cycle of a first feeding session. DPV(MER$_{1,1}$) is thus a duct parameter value from the first MER cycle of the first feeding session. At t= 1, where t indicates a breast feeding or milk pumping session, then equation 1 = 0 and thus indicates no clogging. This is because both duct parameter values would be the same. If DPV(MER$_{1,t}$) were to be less than DPV(MER$_{1,1}$) then AC would be greater than 0 and this might indicate duct clogging if the number is above a certain threshold. In other words, if the duct diameter in a later breastfeeding session is found to be smaller than in the first session (t= 1) then this might indicate duct clogging.

Essentially, a duct geometry or milk flow parameter is measured in an MER cycle of a first breastfeeding or milk pumping session, and this may be used to compare with later measurements during later sessions to determine clogging. Preferably the MER cycle of a session used is the first MER cycle.

A percentage clogging value may be provided by multiplying AC by 100. If a percentage clogging value over a certain percentage is determined, then an early warning indicator may be displayed to the subject. The threshold percentage may be one of: 20%, 30%, 40%, 50%, 60%, 70%. The risk value may be synonymous with the percentage clogging value, or may be a further derived value based on the percentage clogging value.

In an embodiment, the time derivative or rate of change of AC between two successive sessions, e.g. t=2 and t=3, can be calculated and therefore give information on the dynamics of the system.

$$\Delta AC = \frac{DPV(MER_{1,3}) - DPV(MER_{1,2})}{DPV(MER_{1,1})} \qquad (2)$$

$\Delta AC$ is the change of AC. In an embodiment, MER longitudinal data from different sessions over a number of days or weeks may be collected. Some characteristics of MER cycles, like the number and duration of MER cycles in a breastfeeding or milk expression, are usually consistent. Hence, there may be more variation within a single session than across different sessions, and so this longitudinal data may be used to establish subject-personalized milk flow dynamics/patterns that may be indicative of duct clogging or risk of duct clogging.

As a useful metric, AC can also be divided by the duration of the breastfeeding session. Alternatively, or in addition, the sum of ACs across multiple MER cycles in a single session may also be calculated.

The threshold value of step 320 may also be determined based on at least one calibrated duct parameter value of a different milk duct of the subject. The use of a calibrated duct parameter value of a different milk duct of the subject to help inform the threshold value may allow variation between ducts in a single breast or between breasts to be indicative of milk duct clogging.

The milk duct of the subject may be in a different breast to the different milk duct. The use of a calibrated duct parameter value of a different milk duct of the subject to help inform the threshold value may allow variation between breasts to be indicative of milk duct clogging. For instance, calibrated duct parameter values may be determined for the left and right breasts simultaneously, and this information may be used to help determine whether one breast is more at risk of clogging than the other. Alternatively, calibrated duct parameter values may be determined for the left and right breasts at different times.

In other words, relative comparisons using differential measurements or ratios of duct parameter values may be beneficial. One example embodiment is a relative comparison of calibrated duct parameter values of two ducts of the same breast at a fixed/single time point and fixed location. Another example embodiment is a relative comparison of calibrated duct parameter values of one duct from a left and right breast respectively, at a fixed/single time point and fixed location.

For instance, in the example embodiment in which ducts from a left and right breast are being compared, the duct parameter value may preferably be the duct diameter or flow difference of the leading edge of the first MER cycle (although the second or third cycle may also be used). The comparison of these calibrated physiological functions relative to one another can be used as in indicator for duct clogging. The leading edge of the duct diameter, i.e. from point 210 to point 220 in Fig. 2, is proposed to take into account different starting diameters and elasticity properties of various ducts present in a breast and provide a measure of duct compliance. The two or more ducts can be from the same breast or from both as the MER cycle may occur simultaneously in the left and right breasts.

For example, a relative level of duct clogging, $\Delta DC$, can be expressed mathematically as a function of the leading edge changes $\Delta P1$ and $\Delta P2$ of the two duct diameters at the first MER cycle respectively as follows:

$$\Delta DC = \Delta P1(MER_{1,t}) - \Delta P2(MER_{1,t}) \qquad (3)$$

As described above, t indicates a number of the breast feeding or milk pumping session, i.e., the subject's first, second, or third, etc. The 1 indicates that it is the first MER cycle of the session. Using equation 3, $\Delta DC = 0$ thus indicates no relative clogging as the leading edge of the diameter of both milk ducts is identical. If $\Delta DC < 0$, this indicates that duct 1 is prone to clogging, and if $\Delta DC > 0$, this indicates that duct 2 is prone to clogging.

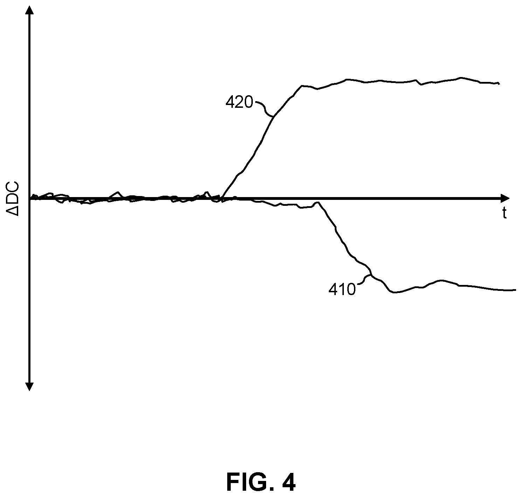

Referring now to Fig. 4, there is depicted a diagram showing the progression of relative levels of duct clogging, $\Delta DC$, for two milk ducts of a subject, 410 and 420 respectively. In this way, a sigmoid-shaped curve or step function can be used to identify the duct prone to clogging. For instance, after $\Delta DC$ for duct 420 has moved or stepped significantly away from 0, this may indicate that the duct is prone to clogging.

One concern is that not all feeding sessions show exactly the same strengths of MER cycles, whereby there will be noise attached to all absolute measurements of $\Delta P$, and by definition, $\Delta DC$. Whilst these effects will be diminished by longitudinal measurement, the inventors have realised that by taking ratios of $\Delta P$ from different ducts at approximately the same time period during an MER cycle or feeding session, the effect of different MER cycle strengths is reduced.

An alternative metric to determine level of duct clogging is as follows:

$$R = \frac{\Delta P1(MER_{1,t})}{\Delta P2(MER_{1,t})} \qquad (4)$$

In equation 4, R stands for ratio. R should be relatively constant, fluctuating around a constant value, i.e., 1 if the leading edge changes of duct diameters 1 and 2 are identical. $R = 1$ indicates both ducts are intact and there is no relative clogging. $R < 1$ indicates duct 1 is prone to clogging. $R > 1$ indicates duct 2 is prone to clogging.

If using metric R, ratios of $\Delta P$ for many ducts may be recorded prior to the occurrences of any blockages. The simultaneous recording of $\Delta P$ for any 2 ducts will thus be able to leverage these past ratios in order to determine whether either of the two current ducts are blocked, as the ratio would be different.

The method 300 may further comprise the step (not shown) of generating a display control signal, the display control signal describing the determined risk. This may provide a way of presenting to the subject the risk of one of their milk ducts being clogged. For instance, the display control signal may comprise feedback that the nipple most recently measured is clogged or at risk of clogging. In another example, the display control signal may comprise feedback indicating an area at which a milk blocked duct has been identified. This may enable the subject to take corrective curative action. The display control signal may comprise a presentation of a graph indicative of a MER cycle of the subject. The display control signal may comprise a graph indicative of a change of a calibrated duct parameter value over time, for instance, over several weeks.

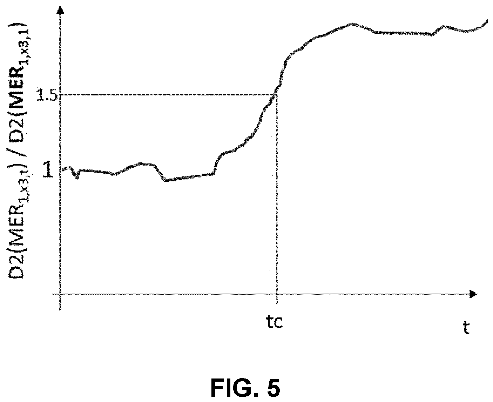

Referring now to Fig. 5, there is provided a graph showing progressive dilation of duct lumen behind a clogged duct at a fixed position. In some embodiments, scanned image stacks at the moment of MER may be analysed to detect abnormal duct dilation behind a blocked duct. In this embodiment, measurements for a single duct are calibrated, and the duct parameter values are determined at varying locations along the duct. In other words, single duct calibration is performed at a varying location, x, along a single duct at either a fixed time point, t, or as a function of time. In this embodiment, either one image at the moment of MER is used, or a sequence of laterally or vertically scanned images at the moment of MER. These images can then be analysed to see whether there is an excessive duct diameter increase behind a blocked duct. In other words, if the duct is clogged then the milk will not be able to be expelled and the duct will expand and swell behind the blockage. Therefore, behind a blocked duct there will be a progressive dilation of duct lumen. For example, as shown in Fig. 5, a duct dilation measurement could be calibrated and if it exceeded 150% of an initial diameter measured at a first MER cycle at a time point, tc, an indication of clogging could be provided.

To enable calibration and the reliable monitoring of changes in duct dilation as a function of position x along a duct in relation to the moment of the first MER cycle of a first feed session, the following equation can be used, with x being the position along the duct (depth-wise), and t being the number of the breastfeeding session:

$$\frac{DPV(MER_{1,x,t})}{DPV(MER_{1,x,1})} \qquad (5)$$

If there was no build-up of duct dilation behind a clogged duct, the graph line shown in Fig. 5, would fluctuate around a value of 1, as the duct diameters would be roughly constant.

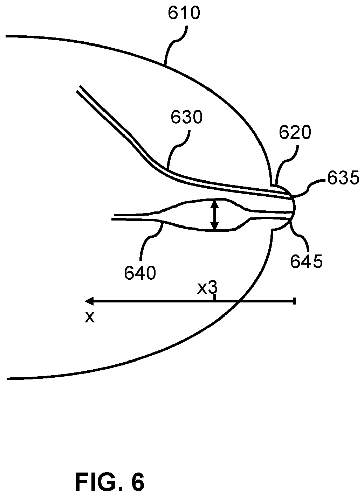

Referring now to Fig. 6, there is depicted a diagram showing dilation of a duct lumen 640 behind a clogged duct 645. There is shown a breast 610 of a subject, and two ducts, 635 and 645, within a nipple 620. The duct 635 is not blocked and therefore the duct lumen 630 of this duct is not dilated. The duct 645, however, is blocked therefore leading to a dilation of the duct lumen 640. A position along the duct lumens from the surface of the nipple into the breast 610 can be defined along axis x.

At a particular position $x=x3$ along this axis, and for a particular duct diameter D2 for duct lumen 640, equation 5 can be re-written as:

$$\frac{D2(MER_{1,x3,t})}{D2(MER_{1,x3,1})} \qquad\qquad (6)$$

Equation 6 may thus be used to monitor/identify abnormal duct dilation over time, and may be plotted as in Fig. 4. As *a priori*, one does not know at which position x to look, equation 5 or 6 should therefore preferably be monitored for a range of positions, $x_i$, with i being the index number of a certain position. A blockage may then be detected if a maximum value of equation 5 or 6 for a range of positions exceeds a threshold. For example, a blockage may be detected using the following equation:

$$MAX_{xi}\left(\frac{DPV(MER_{1,xi,t})}{DPV(MER_{1,xi,1})}\right) > \text{threshold} \qquad\qquad (7)$$

Equation 7 is therefore simply stating that if a maximum value of equation 5 along x exceeds a threshold, then this may indicate a blockage. Alternatively, instead of a maximum, one may look at a certain highest percentile of equations 5 or 6 for a range of positions exceeding a threshold. For example, if 10% of equations 5 or 6 for a range of positions exceed a threshold, or if the standard division of equations 5 or 6 for a range of positions exceeds a threshold. This may then provide a more robust assessment than just considering one maximum value. This embodiment thus provides an advantageous method for determining possible duct clogging by greatly reducing the relevant image data volume needed to analysis, i.e. only analysing images taken at the moment of MER.

In some embodiments, there is provided a computer program comprising code means for implementing any of the methods described above when said program is run on a processing system.

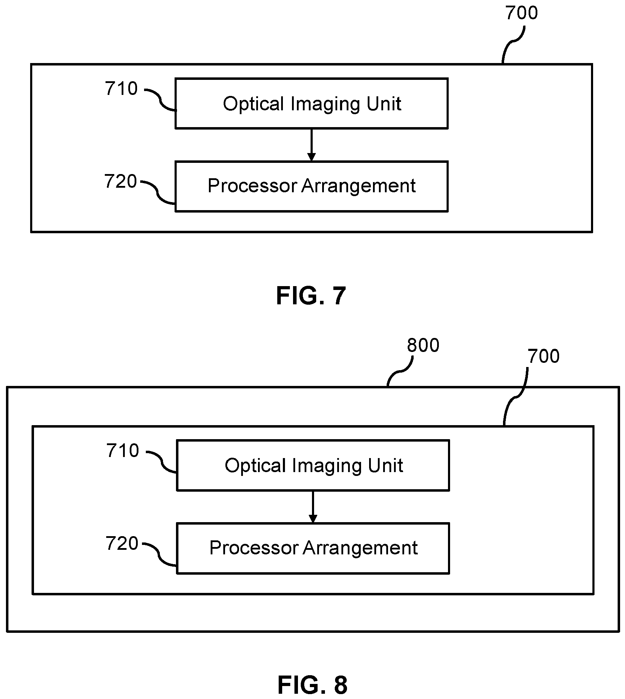

Referring now to Fig. 7, there is depicted a simplified block diagram of a system 700 for calibrating a duct parameter measurement of a milk duct of a subject according to a proposed embodiment.

The system 700 configured for calibrating a duct parameter measurement of a milk duct of a subject comprises an optical imaging unit 710, and a processor arrangement 720. The system 700 is configured to calibrate a duct parameter measurement of a milk duct of a subject by processing detected milk ejection reflex parameter values and determined duct parameter values of a milk duct of the subject.

The optical imaging unit 710 is configured to obtain measurements of the at least one MER cycle of the subject, wherein detecting the at least one MER parameter value is based on the obtained measurements. It is not essential that the system 700 have an optical imaging unit 710, as the system 700 may receive information about at least one MER cycle of the subject from a stand-alone unit, such as a stand-alone optical imaging unit or from a bottle unit that, for example, monitors the rate of filling. However, integrating an optical imaging unit 710 into the system 700 may provide a comprehensive system 700 capable of carrying out any herein disclosed method, such as method 100 or method 300.

In some embodiments, the optical imaging unit 710 may be rotatable and configured to perform a scan of different imaging directions. This may enable a more complete 3D image of a milk duct to be formed. The light source for the optical imaging unit 710 may comprise a near infra red light source. Infra red light may have sufficiently low scattering and absorption to penetrate the nipple fully. Based on scattering differences between milk and tissue a visual contrast between ducts and the surrounding tissue is obtained. Specific wavelengths may be chosen which have a difference in absorption between the milk and the breast/nipple tissue, for example using absorption of fatty acids in milk or other milk specific components.

The optical imaging unit 710 may provide images of a nipple of the subject and, optionally, also a portion of the breast behind the nipple or areola.

The processor arrangement 720 is configured to: detect at least one milk ejection reflect (MER) parameter value of at least one MER cycle of the subject; determine at least one duct parameter value of a milk duct of the subject; and calibrate the determined at least one duct parameter value based on the detected at least one MER parameter value to generate at least one calibrated duct parameter value.

Referring now to Fig. 8, there is depicted a simplified block diagram of a breast pump 800 comprising a system 700 for calibrating a duct parameter measurement of a milk duct of a subject according to a proposed embodiment. A breast pump 800 (not shown in detail) is thereby provided that is capable of optically imaging milk ducts of subjects to obtain measurements and also capable of analysing the measurements to calibrate them and potentially determine a risk of clogging. The breast pump is not shown since no further modification is needed to a standard breast pump design. The breast pump may be wearable or it may be external.

In other words, the system 700 of Fig. 7 may be built into already existing devices or products used by breastfeeding mother, such as a standard breast pump, wearable breast pump or a nipple shield. This has the advantage that in all cases a fluctuating pressure cycle is already present (generated by the breast pump or by the baby sucking) and the mother does not need to spend any additional time performing the detection.

The system of 700 or 800 is intended to be used repeatedly over time, e.g., every day. In this way, a sudden behavior change of a duct is the most reliable signal that blockage is occurring. Thus, each duct will have its own geometry when open and when clogged, and it is the change from one state to the other that is to be detected. To follow individual ducts over time an algorithm may be utilized that is self-learning to recognize the ducts from specific features such as shape or size features, or using neural network algorithms.

Tracking duct geometry over time, e.g., from consecutive pumping sessions over consecutive days, can additionally be a powerful way to decide whether a duct is blocked. Each duct will have its own diameter and diameter fluctuation pattern and also have different unique features such as location in the breast, widening and narrowing patterns, branching patterns etc. Therefore, individual ducts may be recognized and followed over time. If the dynamic movement of a particular duct significantly drops, this may indicate (partial) blockage, and the mother may be alerted to treat it. An algorithm may be utilized capable of learning features, such as a neural network, to improve recognition of individual ducts.

A feedback system may also be provided indicating the areas to which attention is needed, so the mother can for example focus on this area by changing position of breastfeeding, feeding more frequently etc. This may be implemented as a wireless communication to a subject's device such as a smart phone, to provide a report which includes a graphical representation of the breast so that the area of blocked milk duct can be identified. Additionally, there may be alarm features such that the device automatically sends messages, blinking lights or certain sounds etc., to alarm the breastfeeding mother if clogged ducts are detected.

It should be noted that any herein disclosed processing does not need to be in real time. Images can for example be stored and analyzed after the breast pumping or breast feeding event, and the output may be given a short time afterwards. However, real time processing is also possible.

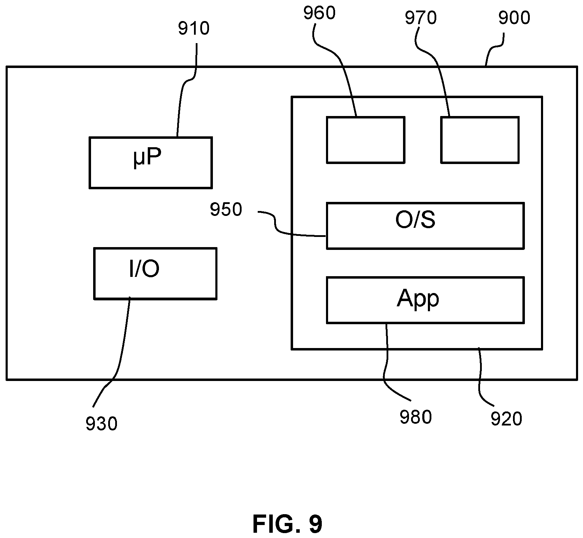

Fig. 9 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g. connected via internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 800 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3, and the systems of Figs. 7 and 8, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 7 or 8 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method for calibrating a duct parameter measurement of a milk duct of a breast of a subject, the method comprising:

    detecting at least one milk ejection reflex (MER) parameter value of at least one MER cycle of the subject;
    determining at least one duct parameter value of a milk duct of the subject; and
    calibrating the determined at least one duct parameter value based on the detected at least one MER parameter value to generate at least one calibrated duct parameter value.

2. The method of claim 1, wherein the at least one MER parameter value comprises at least one of: the peak of the MER cycle; the duration of the MER cycle; whether the MER cycle is the first in a breastfeeding or milk expression session; the start of the MER cycle; the end of the MER cycle; the duration from the start to the peak of the MER cycle; the duration from the peak to the end of the MER cycle.

3. The method of any of claims 1 or 2, wherein detecting at least one MER parameter value of at least one MER cycle of the subject comprises obtaining measurements of the at least one MER cycle of the subject from an optical imaging system.

4. The method of claim 3, wherein determining the at least one duct parameter value is based on the obtained measurements of the at least one MER cycle of the subject.

5. The method of any of claims 1 to 4, wherein the at least one duct parameter value comprises at least one of: milk flow rate; maximum milk flow rate; milk flow volume; total milk volume; duct diameter; change of duct diameter; rate of change of duct diameter; rate of change of duct diameter from the start to the peak of the MER cycle; duct volume; change of duct volume; and rate of change of duct volume.

6. The method of any of claims 1 to 5, wherein the at least one calibrated duct parameter value is indicative of at least a partial clogging of the milk duct of the subject.

7. The method of claim 6, wherein the method further comprises:
    determining a risk value indicating a risk of the milk duct of the subject clogging based on the at least one calibrated duct parameter value.

8. The method of claim 7, wherein determining a risk value comprises comparing the at least one calibrated duct parameter value to a threshold value, and determining the risk value based on the comparison result.

9. The method of claim 8, wherein the threshold value is determined based on at least one past calibrated duct parameter value of the subject's milk duct.

10. The method of claim 8, wherein the threshold value is determined based on at least one calibrated duct parameter value of a different milk duct of the subject.

11. The method of claim 10, wherein the milk duct of the subject is in a different breast to the different milk duct.

12. A computer program comprising code means for implementing the method of any preceding claim when said program is run on a processing system.

13. A system for detecting risk of milk duct clogging, the system comprising:
a processor arrangement configured to:

detect at least one milk ejection reflex (MER) parameter value of at least one MER cycle of the subject;
determine at least one duct parameter value of a milk duct of a breast of the subject; and calibrate the determined at least one duct parameter value based on the detected at least one MER parameter value to generate at least one calibrated duct parameter value.

14. Wherein the system further comprises an optical imaging unit, configured to obtain measurements of the at least one MER cycle of the subject, wherein detecting the at least one MER parameter value is based on the obtained measurements.

15. A breast pump comprising the system of claim 13 or 14.

100

```
┌─────────────────────────┐
│  Detecting a MER        │
│  parameter value of a MER│
│  cycle                   │
│                          │
│  110                     │
└─────────────────────────┘
             │
             ▼
┌─────────────────────────┐
│  Determining a duct     │
│  parameter value of a milk│
│  duct                   │
│                          │
│  120                     │
└─────────────────────────┘
             │
             ▼
┌─────────────────────────┐
│  Calibrating duct parameter│
│  value based on MER     │
│  parameter value        │
│                          │
│  130                     │
└─────────────────────────┘
```

## FIG. 1

FIG. 2

300

310 — Obtaining measurements of MER cycle of subject

110 — Detecting a MER parameter value of a MER cycle

120 — Determining a duct parameter value of a milk duct

130 — Calibrating duct parameter value based on MER parameter value

320 — Comparing calibrated duct parameter value to a threshold value

330 — Determining risk of milk duct clogging based on the comparison

**FIG. 3**

**FIG. 4**

**FIG. 5**

**FIG. 6**

700

710 — Optical Imaging Unit

720 — Processor Arrangement

**FIG. 7**

800

700

710 — Optical Imaging Unit

720 — Processor Arrangement

**FIG. 8**

**FIG. 9**

Europäisches
Patentamt

European
Patent Office

Office européen
des brevets

# EUROPEAN SEARCH REPORT

Application Number

EP 23 16 3671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 827 737 A1 (KONINKLIJKE PHILIPS NV [NL]) 2 June 2021 (2021-06-02) * abstract * * paragraph [0047] * * paragraph [0055] * ----- | 1-15 | INV.<br>A61B5/00<br>G01F1/663<br><br>ADD.<br>A61B8/08 |
| A | EP 1 827 210 A1 (MAMSENSE LTD [IL]) 5 September 2007 (2007-09-05) * abstract * ----- | 1-15 | |

TECHNICAL FIELDS
SEARCHED        (IPC)

A61B
A61M
G01F

The present search report has been drawn up for all claims

| Place of search | Date of completion of the search | Examiner |
|---|---|---|
| The Hague | 8 August 2023 | Dhondt, Erik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 16 3671

This annex lists the patent family members relating to the patent documents cited in the above-mentioned European search report.
The members are as contained in the European Patent Office EDP file on
The European Patent Office is in no way liable for these particulars which are merely given for the purpose of information.

08-08-2023

| Patent document cited in search report | | | Publication date | Patent family member(s) | | | Publication date |
|---|---|---|---|---|---|---|---|
| EP 3827737 | A1 | | 02-06-2021 | AU | 2020393248 | A1 | 14-07-2022 |
| | | | | CA | 3162590 | A1 | 03-06-2021 |
| | | | | CN | 114765945 | A | 19-07-2022 |
| | | | | EP | 3827737 | A1 | 02-06-2021 |
| | | | | EP | 4064960 | A1 | 05-10-2022 |
| | | | | US | 2022395218 | A1 | 15-12-2022 |
| | | | | WO | 2021104858 | A1 | 03-06-2021 |
| EP 1827210 | A1 | | 05-09-2007 | AU | 2005305439 | A1 | 26-05-2006 |
| | | | | CA | 2586644 | A1 | 26-05-2006 |
| | | | | EP | 1827210 | A1 | 05-09-2007 |
| | | | | IL | 165289 | A | 31-03-2014 |
| | | | | JP | 4865723 | B2 | 01-02-2012 |
| | | | | JP | 2008520329 | A | 19-06-2008 |
| | | | | US | 2009054771 | A1 | 26-02-2009 |
| | | | | WO | 2006054287 | A1 | 26-05-2006 |

EPO FORM P0459

For more details about this annex : see Official Journal of the European Patent Office, No. 12/82